# EUROPÄISCHE PATENTANMELDUNG

(11) **EP 2 687 192 A1**
(43) Veröffentlichungstag der Anmeldung: **22.01.2014**
(21) Anmeldenummer: 12176779.2
(22) Anmeldetag: 17.07.2012
(51) Int. Cl.: A61F 7/03, A43B 7/02

(54) **Heizpackung, insbesondere zur Wärmung von Körperteilen**

(71) Anmelder: Eidgenössische Materialprüfungs- und Forschungsanstalt EMPA, 8600 Dübendorf (CH)
(72) Erfinder: Die Erfindernennung liegt noch nicht vor
(74) Vertreter: Schmauder & Partner AG Patent- & Markenanwälte VSP

(57) **Zusammenfassung**

Eine Heizpackung (2) umfasst mindestens ein flächiges Heizelement (4) sowie eine Umhüllung (6), in welcher das Heizelement untergebracht ist. Das Heizelement umfasst mindestens eine wasserdampfdurchlässige Trägerlage (8), welche eine Vielzahl von Zeolithpartikeln (10) enthält, wobei sich die Trägerlage in einem getrockneten Zustand befindet, und wobei die Umhüllung aus einem feuchtigkeitsdichten Material gebildet ist. Die Heizpackung ist insbesondere zur Wärmung oder Warmhaltung von Körperteilen, beispielsweise durch Beheizung eines Schuhs, geeignet.

## Beschreibung

### Technisches Gebiet

Die vorliegende Erfindung betrifft eine Heizpackung gemäss Oberbegriff des Anspruchs 1 sowie die Verwendung einer getrockneten wasserdampfdurchlässigen Lage, welche eine Vielzahl von getrockneten Zeolithpartikeln enthält.

### Stand der Technik

In zahlreichen Situationen, beispielsweise bei kalter Witterung oder bei der Arbeit in einer gekühlten Umgebung, besteht das Bedürfnis nach einer aktiven Wärmung der betroffenen Körperteile bzw. -regionen. Hierfür gibt es bereits verschiedene Lösungsansätze, die wenigstens teilweise auch in der Patentliteratur beschrieben sind.

Beispielsweise wurden schon mehrfache Varianten elektrisch beheizbarer Schuhe vorgeschlagen, die im Sohlenbereich mit Widerstandsdrähten ausgestattet sind und zudem eine entsprechende Stromquelle aufweisen. Ein Beispiel hierfür ist die DE 3904603 A1. Nachteilig an einer elektrischen Heizung ist einerseits, dass für eine mehrstündige Einsatzdauer ein vergleichsweise sperriges und gewichtiges Batterie- oder Akkupack erforderlich ist. Darüber hinaus ist bekannt, dass die Leistungsfähigkeit derartiger Stromquellen gerade in kalten Umgebungen stark beeinträchtigt sein kann.

Schon vor geraumer Zeit wurden deshalb nicht-elektrische Heizlösungen vorgeschlagen. Beispielsweise beschreibt GB 2 361 169 A eine Heizpackung in Form einer Innensohle, deren Heizprinzip auf der beim Auskristallisieren einer übersättigten wässrigen Salzlösung wie z.B. Natriumacetat freigesetzten Wärme beruht. Der Heizvorgang kann dabei in bekannter Art und Weise durch Verbiegen eines kleinen Metallblechs ausgelöst werden. Die Heizpackung kann in der Folge durch Aufheizen in kochendem Wasser und vollständigem Auflösen des Kristallisates aktiviert werden und steht somit für den nächsten Einsatz bereit.

Dank der kompakten Bauweise sind derartige Heizvorrichtungen auch für andere Anwendungsbereiche, beispielsweise als Handwärmer oder generell als situativ einsetzbare Wärmepakete verwendbar. So beschreibt GB 2 411 098 A einen ebenfalls mit einer übersättigten Natriumacetatlösung befüllten taschenförmigen Körperwärmer.

In manchen Situationen sind allerdings noch leichtere, vorzugsweise nicht mit einer Flüssigkeit befüllten Heizpackungen erwünscht, die insbesondere auch für einen sinnvollen Einmalgebrauch geeignet wären. Ein Beispiel hierfür sind die von der Firma The Heat Company GmbH, Altenmarkt (AT) angebotenen Hand- bzw. Fusswärmer, deren Heizprinzip auf einem Oxidationsprozess basiert. Es ist bekannt, dass Vermiculit zusammen mit einer Reihe von Additiven (Eisenpulver, Wasser, Cellulose, Salz und Aktivkohle) zu einer reaktiven Mischung verarbeitet werden kann, die beim Kontakt mit Luftsauerstoff einen exothermen Oxidationsprozess erfährt. Dementsprechend wird der Heizvorgang der besagten Wärmer durch Aufreissen von deren Schutzhülle ausgelöst.

Nachteilig an den oxidationsbasierten Wärmeelementen ist allerdings, dass die entstehenden Temperaturen je nach Umgebungsbedingungen unterschiedlich sind und teilweise bis zu 75°C erreichen.

### Darstellung der Erfindung

Eine Aufgabe der Erfindung ist es demnach, eine verbesserte Heizpackung, die insbesondere zur Wärmung von Körperteilen geeignet ist, bereitzustellen.

Gelöst wird diese Aufgabe durch die im Anspruch 1 definierte Heizpackung. Diese umfasst erfindungsgemäss mindestens ein flächiges Heizelement sowie eine Umhüllung, in welcher das Heizelement untergebracht ist. Das Heizelement umfasst mindestens eine wasserdampfdurchlässige Trägerlage, welche eine Vielzahl von Zeolithpartikeln enthält, wobei sich die Trägerlage in einem getrockneten Zustand befindet, und wobei die Umhüllung aus einem feuchtigkeitsdichten Material gebildet ist.

Es wurde gefunden, dass in einer geeigneten wasserdampfdurchlässigen Trägerlage eingebettete Zeolithpartikel in getrocknetem, also weitgehend entwässertem Zustand bereitgestellt werden können und dass diese beim Kontakt mit Luftfeuchtigkeit erhebliche Wärmemengen abgeben.

Ein weiterer Vorteil der erfindungsgemässen Heizpackung ist dadurch gegeben, dass sie während des Heizvorgangs überdies die vom Körper produzierte Feuchtigkeit aufnimmt und somit nicht nur eine warme, sondern auch eine trockene Umgebung zu schaffen vermag.

Die erfindungsgemässe Heizpackung zeichnet sich durch eine kompakte und leichtgewichtige Ausgestaltung, durch eine einfache und zuverlässige Anwendbarkeit sowie durch eine kostengünstige Herstellbarkeit aus. Aufgrund der verwendbaren Materialien ist eine ökologisch vertretbare Entsorgung möglich, was die Verwendung als Einwegartikel ermöglicht.

Eine weitere Aufgabe der Erfindung ist darin zu sehen, eine Verwendung für eine getrocknete wasserdampfdurchlässige Lage, welche eine Vielzahl von Zeolithpartikeln enthält, anzugeben.

Diese weitere Aufgabe wird durch die im Anspruch 8 definierte Verwendung als ein durch Luftfeuchtigkeit aktivierbares flächiges Heizelement gelöst.

Vorteilhafte Ausgestaltungen der Erfindung sind nachfolgend beschrieben und in den abhängigen Ansprüchen definiert.

Der Begriff "Zeolith" ist an sich bekannt und beispielsweise im Römpp Chemie Lexikon, 9. erweiterte und neubearbeitete Auflage, Georg Thieme Verlag, Stuttgart, New York (1992), Band T-Z, S. 5119 - 5121 definiert. Unter dem Begriff "Zeolithpartikel" sind im vorliegenden Zusammenhang grundsätzlich jegliche Partikel aus irgendeinem Zeolithen zu verstehen. Hierbei kann es sich um grobe Körner mit einer Grösse im Bereich von Millimetern bis hinunter zu sehr feinen, mikrokristallinen Körnern im Bereich von Mikrometern handeln. Insbesondere umfasst der Begriff "Zeolithpartikel" auch kalzinierte Zeolithkörner, welche zahlreiche, durch einen Binder aggregierte Zeolith-Mikrokristalle enthalten.

Unter dem Begriff "flächiges Heizelement" ist im vorliegenden Zusammenhang ein Heizelement abgeflachter Form zu verstehen, welches also in einer Richtung senkrecht zu einer Hauptebene der besagten Form eine vergleichsweise geringe Dicke aufweist. Ein derart geformtes Heizelement eignet sich gut zur Wärmeübertragung auf eine Empfängerfläche wie beispielsweise die Hand- oder Fussfläche einer Person.

Unter dem Begriff "wasserdampfdurchlässige Trägerlage" ist im vorliegenden Zusammenhang eine Lage zu verstehen, welche einen guten Zugang von Umgebungsluft zu allen Regionen innerhalb der Trägerlage erlaubt und damit insbesondere die Zufuhr feuchter Umgebungsluft zu den in der Trägerlage eingebetteten Zeolithpartikeln erlaubt.

Unter dem Begriff "feuchtigkeitsdichtes Material" ist im vorliegenden Zusammenhang jegliches Material zu verstehen, die im Wesentlichen wasserdampfundurchlässig ist und somit erlaubt, die getrocknete Trägerlage vor einem ungewollten Zutritt von Luftfeuchtigkeit zu bewahren. Vorzugsweise handelt es sich dabei um eine feuchtigkeitsdichte Folie. Beispielsweise kann hierfür eine aus der Lebensmittelindustrie bekannte mehrschichtige Polypropylenfolie verwendet werden. Es könnten aber auch metallbeschichtete Kunststofffolien eingesetzt werden.

Der Begriff "getrockneter Zustand" der Trägerlage ist dahingehend zu verstehen, dass sich die Trägerlage einschliesslich der darin eingebetteten Zeolithpartikel in einem Zustand befinden wie er zum Beispiel nach Aufbewahrung und Äquilibrierung in einem Feinvakuum von < 1 hPa bei 100°C erreicht wird. Dies lässt sich beispielsweise in einem herkömmlichen Vakuum-Trockenschrank erreichen. Ohne Vakuum sind entsprechend höhere Temperaturen für die Trocknung notwendig.

Durch Variieren folgender Parameter ist es möglich, die Kinetik der Wasseraufnahme sowie die Dauer der Wärmeabgabe anzupassen:
- Matrixmaterial (verschiedene Wasser(dampf)durchlässigkeit)
- Dimension und Design der Matrix (Film, Faser, Schaum, Mikrokanäle,...)
- Zeolithtyp
- Zeolithform (Granulat, Extrudat, Pulver)
- Verteilung des Zeoliths in der Matrix
- Zeolithmenge

Nach Ende der Wirkung kann das Material entsorgt oder mehrmals im Ofen oder in einem handelsüblichen Mikrowellengerät reaktiviert werden. Wird das Material wasserdicht verpackt (z.B. in handelsüblichen, verschliessbaren Plastikbeuteln), kann es während mehrerer Wochen/Monate einsatzfähig erhalten werden.

Grundsätzlich kann die wasserdampfdurchlässige Trägerlage aus einer Vielzahl poröser oder fasriger Materialien ausgewählt werden. Beispielsweise kann sie aus einem Vliesstoff (engl. "Non-Woven") oder einem anderen textilen Gebilde hergestellt werden.

Grundsätzlich sind die Abmessungen der Trägerlage keinen besonderen Einschränkungen unterworfen und können demnach entsprechend dem gewünschten Verwendungszweck gewählt werden. Typischerweise kann die Trägerlage eine Dicke von 0.1 mm bis 10 mm, insbesondere von 2 mm bis 4 mm aufweisen. Dabei können Zuschnitte der gewünschten Form und Flächengrösse, beispielsweise ab ungefähr 1 cm² verwendet werden. Für Anwendungen im Bekleidungsbereich können Zuschnitte von beispielsweise 15 bis 600 cm² bereitgestellt werden, während für gewisse Anwendungen wie in Schlafsäcken Zuschnitte bis zu 3 m² vorgesehen werden können.

Gemäss einer besonders bevorzugten Ausgestaltung (Anspruch 2) ist die Trägerlage aus einem cellulosebasierten Material wie beispielsweise Zellstoff gebildet. Vorteilhaft ist dabei, dass sich auf kostengünstigem Wege eine leichtgewichtige, genügend stabile, flexible und gut entsorgbare Trägerlage herstellen lässt. Insbesondere kann die Trägerlage aus Papier gebildet sein (Anspruch 3). In diesem Fall liegt ein weiterer Vorteil darin, dass das getrocknete Papier selber auch Energie abgeben kann, wenn es nach Trocknung Wasserdampf absorbiert und so den Effekt der Zeolithpartikel verstärkt.

Der Anteil an Zeolithpartikeln ist entsprechend dem vorgesehenen Einsatzbereich zu wählen. Dabei weist die Trägerlage ein Gewichtsverhältnis von Zeolithpartikeln zu Papier von 0.1 bis 2, vorzugsweise 0.5 bis 1.5, insbesondere 0.8 bis 1.0 auf (Anspruch 4).

Gemäss einer weiteren Ausgestaltung (Anspruch 5) ist die Trägerlage aus einer offenporigen oder/und wasserdampfdurchlässigen Polymermatrix, beispielsweise aus einem zumindest teilweise offenporigen Polyurethanschaum, gebildet.

Gemäss einer weiteren Ausgestaltung (Anspruch 5) kann für die Trägerlage ein wasserdampfdurchlässiges Polymer wie beispielsweise Sympatex^{®} gewählt werden. Hierbei handelt es sich um ein an sich bekanntes Blockcopolymer aus Polyester und Polyether. Zeolithpartikel könnten hier sandwichartig zwischen Sympatex-Folien oder matrixartig in das Polymer eingebaut werden.

Grundsätzlich umfasst die erfindungsgemässe Heizpackung mindestens ein flächiges Heizelement, das in einer aufreissbaren Umhüllung untergebracht ist. Es ist aber auch möglich, mehrere aneinander gereihte flächige Heizelemente vorzusehen. Damit lassen sich vergleichsweise grossflächige Heizpackungen mit guter Biegsamkeit realisieren.

Gemäss einer weiteren Ausgestaltung (Anspruch 6) umfasst ein jedes flächiges Heizelement mehrere flächig aufeinander geschichtete Trägerlagen. Eine solche sandwichartige Anordnung ermöglicht es beispielsweise, Trägerlagen mit unterschiedlichem Zeolithgehalt und/oder unterschiedlichen Zeolith-Typen zu bilden. Beispielsweise kann eine äussere Trägerlage mit schnell ansprechendem Aufheizverhalten vorgesehen werden, an welche eine innere Trägerlage mit langsamer einsetzendem aber länger anhaltendem Aufheizverhalten anschliesst. Erforderlichenfalls können auch komplexere Schichtanordnungen mit zahlreichen Trägerlagen herangezogen werden.

Es versteht sich, dass die feuchtigkeitsdichte Umhüllung zur Aktivierung des Heizlements aufgerissen, aufgestochen oder anderweitig geöffnet werden muss. Bei einer vorteilhaften Ausgestaltung (Anspruch 7) ist die Umhüllung mit einer Aufreisslinie versehen. Beispielsweise kann die Aufreisslinie als vorzugsweise umlaufende Zone mit geringerer Foliendichte ausgestaltet sein.

Gemäss einer bevorzugten Ausführungsform (Anspruch 9) wird das durch Luftfeuchtigkeit aktivierbare flächige Heizelement zur Beheizung eines Schuhs, beispielsweise eine Skischuhs verwendet.

### Wege zur Ausführung der Erfindung

Ausführungsbeispiele der Erfindung werden nachfolgend anhand der Zeichnungen näher beschrieben, dabei zeigen:
- Fig. 1: eine Heizpackung, in Draufsicht;
- Fig. 2: die Heizpackung der Fig. 1, in seitlicher Ansicht;
- Fig. 3: eine weitere Heizpackung, in seitlicher Ansicht;
- Fig. 4: noch eine weitere Heizpackung, in seitlicher Ansicht; und
- Fig. 5: den zeitlichen Verlauf der Temperatur eines Heizelementes nach Exposition mit standardisierter Umgebungsluft (23°C. 50% relative Luftfeuchtigkeit).

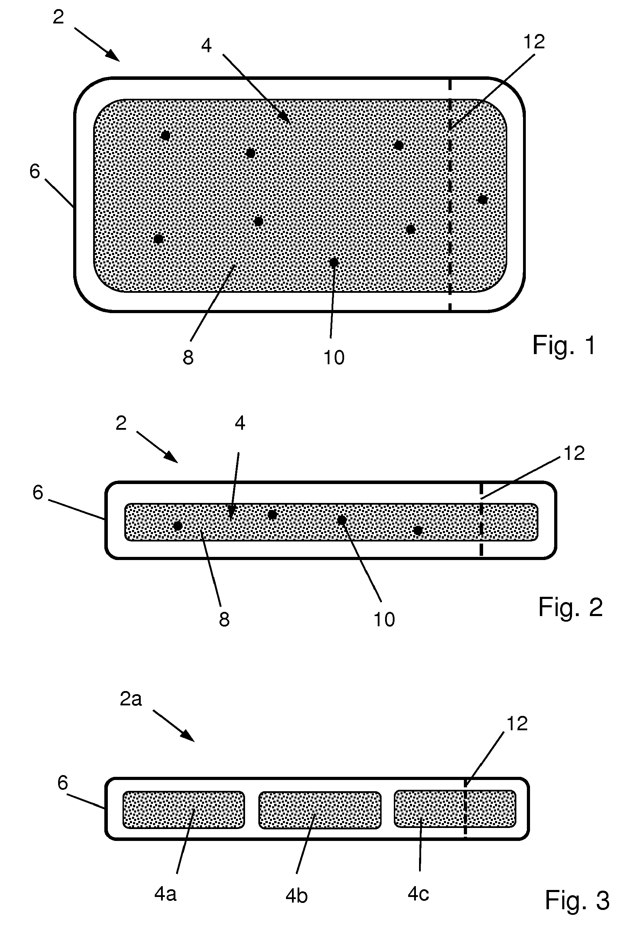

Die in den Fig. 1 und 2 dargestellte Heizpackung 2 umfasst ein flächiges Heizelement 4 sowie eine aufreissbare Umhüllung 6, in welcher das Heizelement untergebracht ist. Das Heizelement 4 ist durch eine wasserdampfdurchlässige Trägerlage 8 gebildet, die eine Vielzahl von Zeolithpartikeln 10 enthält. Letztere sind in den Figuren lediglich schematisch als einzelne wenige Punkte dargestellt.

Die Umhüllung 6 ist aus einer feuchtigkeitsdichten Folie gebildet und weist eine umlaufende Aufreisslinie 12 auf, die beispielsweise als Zone mit geringerer Foliendichte ausgestaltet sein kann. Wesentlich ist jedoch, dass diese Zone wie die übrigen Folienbereiche feuchtigkeitsdicht sein muss. In der hier dargestellten, gebrauchsfertigen Konfektion befinden sich die Trägerlage 8 und insbesondere die darin eingebetteten Zeolithpartikel 10 in einem getrockneten Zustand.

Die in der Fig. 3 dargestellte Heizpackung 2a unterscheidet sich von derjenigen der Fig. 1 und 2 lediglich dadurch, dass sich in der Umhüllung 6 drei im Wesentlichen identische flächige Heizelemente 4a, 4b und 4c befinden. Diese Heizelemente sind aneinander gereiht, d.h. ihre Hauptflächen sind im Wesentlichen koplanar. Grundsätzlich können die einzelnen Heizelemente durch geeignete Fixiermittel aneinander befestigt sein.

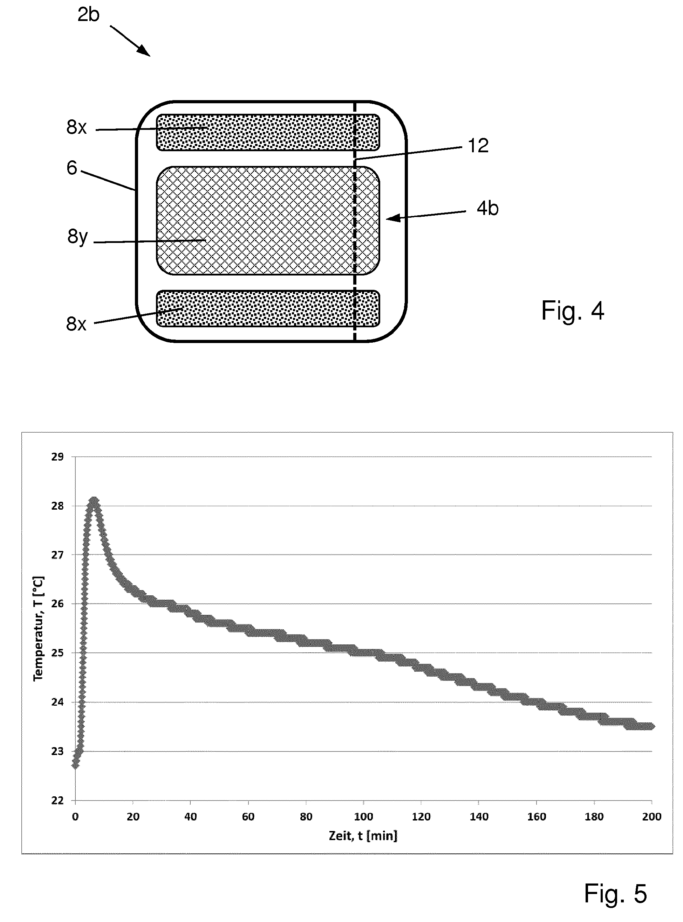

Die in der Fig. 4 dargestellte Heizpackung 2b enthält ein mehrschichtiges Heizelement 4b, das in einer aufreissbaren Umhüllung 6 angeordnet ist. Im gezeigten Beispiel umfasst das mehrschichtige Heizelement drei flächig aufeinander gestapelte Trägerlagen 8x, 8y, 8x. Beispielsweise sind die beiden äusseren Trägerlagen 8x vergleichsweise dünner und mit einem vergleichsweise hohen Anteil an Zeolithpartikeln beladen, wogegen die innere Trägerlage 8y vergleichsweise dicker und mit einem vergleichsweise geringen Anteil an Zeolithpartikeln beladen ist. Die erwähnte Konfiguration stellt jedoch lediglich ein Beispiel zur Erläuterung dar.

### Beispiele

### A) Herstellung einer Pulpe, um Papier zu schöpfen

Zellstoffpapier in Form einer Rolle Toiletten-Papier wird in ca. briefmarkengrosse Stücke zerrissen, welche in einem Eimer mit kochendem Wasser übergossen werden, sodass das Papier gut bedeckt ist. Anschliessend wird die Masse kräftig gerührt/gemixt. Nachdem die Papierstücke 24 h lang eingeweicht wurden, wird die Masse nochmals mit einem Stabmixer gemixt. Von der resultierenden Papiermasse (Pulpe) wird 1 Teil entnommen und 2 Teile Wasser dazu gefügt. Danach wird das Ganze nochmals gut gemixt. Die so gewonnene Pulpe wäre nun beispielsweise bereit, um herkömmliches Papier zu schöpfen. Dies kann mit einem normalen Sieb bewerkstelligt werden.

### B) Herstellung von Papier mit Zeolith

Um Zeolithpartikel ins Papier einzubauen, wurden zwei Varianten getestet, nämlich eine direkte und eine schichtweise Variante.
- In der direkten Variante wird der Pulpe eine bestimmte Menge eines Zeoliths (z. B. Typ Sapo-34) beigefügt. Das Verhältnis Papier: Zeolith kann im trockenen Zustand z.B. 1:1 betragen. Die Masse muss während des Schöpfprozesses in Bewegung gehalten werden, damit das Zeolith gleichmässig in der Pulpe verteilt bleibt. Beliebige, in der Papierindustrie übliche Additive könnten an dieser Stelle dazu gefügt werden.
- In der schichtweisen Variante wird eine Schicht Papier konventionell hergestellt (ohne Zeolith). Solange das Papier noch nass ist, wird eine definierte Menge Zeolith auf das Papier verteilt. Immer noch in nassem Zustand wird anschliessend eine zweite Schicht Papier über das erste gelegt, sodass eine Art Sandwich mit Zeolith zwischen Papier entsteht. Auch hier wurden mit einem Verhältnis Papier : Zeolith = 1:1 gute Ergebnisse erzielt.
- Die Kombination der direkten und schichtweisen Variante ist auch möglich.

Anschliessend wird das Papier in eine für die vorgesehene Verwendung geeignete Gestalt gepresst und an Luft getrocknet. Um die Zeolithpartikel im Papier zu aktivieren muss das ganze Papier im (Vakuum-)Ofen getrocknet werden.

Es wurden zahlreiche Zeolith-Typen getestet, insbesondere die bei Zeochem AG, Uetikon erhältlichen:
- Sapo 34
- Zeolith 10-01 (13X) Pulver
- Zeolith Z4-04
- Zeolith Z4-01
- Zeolith 5A
- Zeox Z12-07
- Zeolith ZEOcat NaY und
- Zeolith Y.

### C) Wärmeentwicklung in einem Heizelement

Mit den oben beschriebenen Methoden wurden flächige Papier-Trägerlagen mit Zeolithpartikeln des Typs ZEOX 12-07 (Lieferant: Zeochem AG) mit einer Körnung von 0.5 bis 0.8 hergestellt. In den geprüften Mustern betrug der Gewichtsanteil am Zeolith 25%. Die Muster wurden während 2 h bei 100°C in einem Vakuumofen getrocknet und anschliessend in einem Exsikkator aufbewahrt, um auf Raumtemperatur abzukühlen. Die Versuche wurden gestartet, indem die getrockneten Muster einen klimatisierten Raum mit 23°C und 50% relativer Feuchtigkeit (RH) gebracht wurden. Der Temperaturverlauf zwischen zwei Papierstücken wurde aufgezeichnet. Wie aus der Fig. 5 ersichtlich, wurde eine Maximaltemperatur von 28°C erreicht, wobei nach 90 min die Temperatur immer noch über 25°C und damit um 2°C über Raumtemperatur lag.

### D) Schuheinlagesohle mit Zeolith zum Heizen und Entfeuchten für den Einweggebrauch

Heizende Schuheinlagesohlen können als Wegwerfprodukt hergestellt werden, welches aus Zellulose und Zeolith besteht und bei einem Kontakt mit Feuchte Wärme frei setzt. Die Temperaturentwicklung hängt im Wesentlichen von der Kinetik ab, mit welcher Wasser vom Zeolith adsorbiert wird. Grundsätzlich kann eine Temperaturerhöhung von ca. 3 bis 5°C über eine Zeit von 1 bis 4 Stunden erreicht werden (bei 50% relativer Luftfeuchtigkeit). Je nach Zeolithtyp, Zeolithform, Einbettung in die Trägersubstanz (Papier oder Karton) und Zeolithmenge ist eine mehr oder weniger starke Aufheizung über einen mehr oder weniger langen Zeitraum möglich. Ziel ist es, eine Entfeuchtung des Schuhbereichs und gleichzeitig eine moderate Erwärmung zu bewirken. Die aktivierte Schuheinlage (getrocknet im Ofen/ in Vakuum/ Mikrowelle) wird mit einer Plastikfolie zum Schutz vor Feuchteaufnahme verpackt. Solche Sohleneinlagen sollen langsam Feuchtigkeit aufnehmen und Wärme abgeben. Da es in Schuhen meistens zu feucht ist, können diese Sohleneinlagen nicht nur im Winter zum Heizen verwendet werden, sondern sind allgemein auch zum Entfeuchten geeignet. Allerdings sollte die Kinetik des Zeoliths in diesen Fällen so ausgelegt sein, dass eine langsame Speicherung der Fussfeuchte möglich ist und sich die Erwärmung in Grenzen hält. Wird nur Zellulose / Biopolymer und Zeolith eingesetzt, sind die Sohleneinlagen biologisch abbaubar und gut geeignet für den Einmalgebrauch. Durch die Reduktion der relativen Feuchte im Schuh sinkt die Anfälligkeit zur Blasenbildung, überdies vermindert sich die Geruchsbelästigung, da diese direkt von der Feuchte im Schuh abhängig ist und Zeolith in der Lage ist, Geruchsstoffe zu adsorbieren.

### E) Wintersportbekleidung (Handschuheinlagen / Faserpelzjacken (Fleece) / Hautnahe Schichten)

Diese Technologie kann auch im Bereich der aktiven Wintersportbekleidung als hautnahe Schicht am Rumpf eingesetzt werden (wenn geschwitzt wird, aber keine übermässige Kühlung stattfinden soll). Heute gibt es bereits funktionelle Shirts die beim Schwitzen relativ rasch die Feuchte in eine aussen liegende Schicht bringen sollen damit keine unangenehme Verdunstungskühlung auf der Haut entsteht. Dies funktioniert aber nur bedingt und dieser Effekt könnte, mit dem Einsatz der erfindungsgemässen Heizpackungen als wärmenden Superadsorber zwischen zwei Bekleidungsschichten noch deutlich verbessert werden. Bei intermittierenden Aktivitäten im Wintersportbereich kleidet sich der Mensch genau so warm, dass er in Ruhe nicht friert. Sobald er aber eine leichte Anstrengung vollbringt, ist die Gesamtwärmeisolation zu hoch und er beginnt zu schwitzen, was wiederum zu einer Auskühlung aufgrund der Verdunstungskühlung führt. Kurze Zeit nach dem Schwitzpuls steht die Person jedoch meist wieder still und wird aufgrund der Feuchte im System frieren. Genau dieser Effekt kann mit der Spezialunterwäsche aus Zeolith verhindert werden, weil die Feuchte das Zeolith aktiviert und so Wärme freigesetzt wird.

### F) Schlafsack

In analoger Weise können die Heizelemente als flexible Innenschicht eines Schlafsacks konzipiert werden, welche Feuchtigkeit im Schlafsack aufnimmt und Wärme abgibt.

### G) Anpassung des Heizverhaltens

Die Heizleistung wie auch der zeitliche Verlauf der Heizwirkung hängen nicht nur vom verwendeten Zeolith-Typen, sondern auch von weiteren Parametern ab. Dazu zählen insbesondere die Körnigkeit der Zeolithpartikel, wobei gröbere Körner ein vergleichsweise langsames zeitliches Ansprechen haben, sowie der Mengenanteil an Zeolithpartikeln, wobei ein höherer Anteil eine stärkere Heizwirkung mit sich bringt. Der zeitliche Verlauf lässt sich vor allem durch die Feuchtigkeitsdurchlässigkeit des Trägermaterials beeinflussen. Erforderlichenfalls können sandwichartige Strukturen mit mehreren aufeinander gestapelten Trägerlagen verwendet werden, und gewünschtenfalls auch verzögernde Zwischenschichten ohne Zeolith eingelegt werden.

## Patentansprüche

1. Heizpackung (2; 2a; 2b), insbesondere zur Wärmung von Körperteilen, umfassend mindestens ein flächiges Heizelement (4; 4a; 4b) sowie eine Umhüllung (6), in welcher das Heizelement untergebracht ist, **dadurch gekennzeichnet, dass** das Heizelement mindestens eine wasserdampfdurchlässige Trägerlage (8) umfasst, welche eine Vielzahl von Zeolithpartikeln (10) enthält, wobei sich die Trägerlage in einem getrocknetem Zustand befindet, und wobei die Umhüllung aus einem feuchtigkeitsdichten Material gebildet ist.

2. Heizpackung nach Anspruch 1, wobei die Trägerlage aus einem cellulosebasierten Material gebildet ist.

3. Heizpackung nach Anspruch 1, wobei die Trägerlage aus Papier gebildet ist.

4. Heizpackung nach Anspruch 3, wobei in der Trägerlage das Gewichtsverhältnis von Zeolithpartikeln und Papier 0.1 bis 2, vorzugsweise 0.5 bis 1.5, insbesondere 0.8 bis 1.0 beträgt.

5. Heizpackung nach Anspruch 1, wobei die Trägerlage aus einer offenporigen und/oder wasserdampfdurchlässigen Polymermatrix gebildet ist.

6. Heizpackung nach einem der Ansprüche 1 bis 5, wobei das flächige Heizelement (4b) mehrere flächig aufeinander geschichtete Trägerlagen (8x, 8y, 8x) umfasst.

7. Heizpackung nach einem der Ansprüche 1 bis 6, wobei die Umhüllung mit einer Aufreisslinie (12) versehen ist.

8. Verwendung einer getrockneten wasserdampfdurchlässigen Lage, welche eine Vielzahl von Zeolithpartikeln enthält, als ein durch Luftfeuchtigkeit aktivierbares flächiges Heizelement.

9. Verwendung nach Anspruch 8 zur Beheizung eines Schuhs.
